# EUROPEAN PATENT APPLICATION

(11) **EP 4 843 997 A1**
(43) Date of publication of application: **25.11.2026**
(21) Application number: 25803809.0
(22) Date of filing: 09.05.2025
(51) Int. Cl.: C07K 14/34, C12N 15/77, C12P 13/08

(54) **POLYNUCLEOTIDE VARIANT AND METHOD FOR PRODUCING L-VALINE BY USING SAME**

(30) Priority: 14.05.2024 KR 20240063418; 15.10.2024 KR 20240140452
(71) Applicant: CJ Cheiljedang Corporation, Seoul 04560 (KR)
(72) Inventor: KIM, Gyuree, Seoul 04560 (KR); KIM, Bina, Seoul 04560 (KR); YUN, Hyojin, Seoul 04560 (KR); KIM, Eunji, Seoul 04560 (KR); CHOI, Jin-Geun, Seoul 04560 (KR); KANG, Hae-Won, Seoul 04560 (KR); KIM, Hee Ju, Seoul 04560 (KR); KIM, Seon Hye, Seoul 04560 (KR)
(74) Representative: Meissner Bolte Partnerschaft mbB
(86) International application number: PCT/KR2025/006255
(87) International publication number: WO 2025/239610

(57) **Abstract**

The present invention relates to a novel polynucleotide exhibiting promoter activity, and a method for producing L-valine by using same. A microorganism into which the novel polynucleotide, of the present invention, having a mutated specific position of a promoter region of a CEY17_07095 gene, a CEY17_14380 gene, and/or a CEY17_06565 gene has been introduced has a significantly increased ability to produce L-valine, and thus the novel polynucleotide can be effectively used in efficient L-valine production.

## Description

### [TECHNICAL FIELD]

The present disclosure claims the benefit of priority to Korean Patent Application No. 10-2024-0063418, filed on May 14, 2024, and Korean Patent Application No. 10-2024-0140452, filed on October 15, 2024, and the entire contents disclosed in the documents of the corresponding Korean patent applications are incorporated herein by reference in their entirety.

Throughout the present disclosure, numerous papers and patent documents are referenced and citations thereof are indicated. The disclosures of the cited papers and patent documents are incorporated into the present disclosure in their entirety by reference to more clearly describe the level of the technical field to which the present invention pertains and the contents of the present invention.

The present disclosure relates to a novel promoter and a method for producing L-valine using the same, and more particularly, to a novel polynucleotide having a promoter activity, an expression cassette comprising the polynucleotide and a target gene operably linked thereto, a vector comprising the polynucleotide or the expression cassette, a microorganism comprising the polynucleotide or the expression cassette, and a method for producing L-valine using the microorganism.

### [BACKGROUND ART]

To produce target substances, such as amino acids or other useful materials usable for various applications including feed, pharmaceuticals, and food, at high titers using microorganisms, efforts such as genetic manipulation and/or introduction of exogenous genes in the biosynthetic pathway have continuously been made. Among such approaches, one method involves inducing overexpression of a target gene in microorganisms, for which a highly efficient gene expression system is required. Since promoters are key elements that significantly influence the level and regulation of gene expression, the development of useful promoters is essential for establishing such expression systems.

Microorganisms of the genus *Corynebacterium* are known to produce amino acids, including L-valine. However, unlike other industrial microorganisms such as *Escherichia coli* or *Bacillus subtilis*, the general structure of promoter sequences for gene expression in *Corynebacterium* has not been well characterized. Accordingly, promoters have been developed by removing the promoter region of an antibiotic resistance gene, such as that for chloramphenicol, inserting chromosomal DNA fragments derived from *Corynebacterium* that have been digested with appropriate restriction enzymes, transforming *Corynebacterium* with the resulting constructs, and then measuring the antibiotic resistance of the obtained transformants. In addition, various promoters have been explored to achieve overexpression of heterologous genes in microorganisms of the genus *Bacillus*, and such promoters have been used for the production of food-grade, pharmaceutical, and other industrial enzymes. To date, numerous vector systems utilizing promoters for the expression of genes encoding α-amylase, protease, and lipase in various *Bacillus* species have been continuously developed (Schumann, 2007, Adv. Appl. Microbiol., 153:813-821).

With the increasing demand for L-valine, there remains a need to overexpress genes related to L-valine production in microorganisms of the genus *Corynebacterium*, and the development of strong promoters is still required.

### [Prior Art Documents]

### [Patent Documents]

(Patent Document 1) US 8465962 B2

### [DETAILED DESCRIPTION OF THE INVENTION]

### [TECHNICAL PROBLEM]

One aspect of the present disclosure provides a novel polynucleotide.

Another aspect of the present disclosure provides an expression cassette comprising the polynucleotide and a target gene.

Another aspect of the present disclosure provides a microorganism comprising the polynucleotide; or the polynucleotide and a target gene operably linked thereto.

Another aspect of the present disclosure provides a method for producing L-valine, comprising culturing the microorganism in a medium.

Another aspect of the present disclosure provides a use of the microorganism for producing L-valine.

### [TECHNICAL SOLUTION]

A detailed description thereof is as follows. Meanwhile, each description and embodiment disclosed in the present disclosure may also be applied to other descriptions and embodiments. That is, all combinations of various elements disclosed in the present disclosure fall within the scope of the present disclosure. In addition, the scope of the present disclosure should not be considered to be limited by the specific descriptions provided below. Furthermore, those skilled in the art will recognize, or may ascertain using no more than routine experimentation, many equivalents to the specific aspects of the present disclosure described in the present disclosure. In addition, such equivalents are intended to be included in the present disclosure.

Furthermore, throughout the present disclosure, numerous papers and patent documents are referenced and citations thereof are indicated. The disclosures of the cited papers and patent documents are incorporated into the present disclosure in their entirety by reference to more clearly describe the level of the technical field to which the present disclosure pertains and the contents of the present disclosure.

An aspect of the present disclosure provides a polynucleotide, comprising a nucleotide sequence wherein the nucleotide at position 130 is substituted with a nucleotide different from the original nucleotide in the nucleotide sequence of SEQ ID NO: 138,
a nucleotide sequence wherein the nucleotide at position 71 is substituted with a nucleotide different from the original nucleotide in the nucleotide sequence of SEQ ID NO: 139, or
a nucleotide sequence wherein the nucleotide at position 150 is substituted with a nucleotide different from the original nucleotide in the nucleotide sequence of SEQ ID NO: 140.

In the present disclosure, the nucleotide sequence of SEQ ID NO: 138, SEQ ID NO: 139, or SEQ ID NO: 140 may be retrieved in a known database, NCBI Genbank, and the nucleotide sequence of SEQ ID NO: 138, SEQ ID NO: 139, or SEQ ID NO: 140 may each be derived from *Corynebacterium sp.*, and specifically, may be a sequence derived from *Corynebacterium glutamicum*. The nucleotide sequence of SEQ ID NO: 138, SEQ ID NO: 139, or SEQ ID NO: 140 may be an exemplary sequence for specifying a mutation site to be introduced for preparing the polynucleotide of the present disclosure, and it is apparent that the mutation introduced into the polynucleotide of the present disclosure may also be introduced into any sequence that is functionally corresponding to the nucleotide sequence of SEQ ID NO: 138, SEQ ID NO: 139, or SEQ ID NO: 140. In one embodiment, the functionally corresponding sequence may be a nucleotide sequence having at least 70% or more, 80% or more, 85% or more, 90% or more, 95% or more, 96% or more, 97% or more, 98% or more, or 99% or more, and less than 100 homology or identity to the nucleotide sequence of SEQ ID NO: 138, SEQ ID NO: 139, or SEQ ID NO: 140, or a nucleotide sequence having the homology or identity in which some sequences are added, deleted, or modified, but is not limited thereto.

Accordingly, in one embodiment, the polynucleotide of the present disclosure may comprise, consist essentially of, or consist of a nucleotide sequence wherein the nucleotide at position 130 is substituted with a nucleotide different from the original nucleotide in the nucleotide sequence of SEQ ID NO: 138, and which has at least 70% or more, 80% or more, 85% or more, 90% or more, 95% or more, 96% or more, 97% or more, 98% or more, or 99%; and less than 100% homology or identity to the nucleotide sequence of SEQ ID NO: 138, or a nucleotide sequence having the homology or identity in which some sequences are added, deleted, or modified. In another embodiment, the polynucleotide of the present disclosure may comprise, consist essentially of, or consist of a nucleotide sequence wherein the nucleotide at position 71 is substituted with a nucleotide different from the original nucleotide in the nucleotide sequence of SEQ ID NO: 139, and which has at least 70% or more, 80% or more, 85% or more, 90% or more, 95% or more, 96% or more, 97% or more, 98% or more, or 99% or more; and less than 100% homology or identity to the nucleotide sequence of SEQ ID NO: 139, or a nucleotide sequence having the homology or identity in which some sequences are added, deleted, or modified. In another embodiment, the polynucleotide of the present disclosure may comprise, consist essentially of, or consist of a nucleotide sequence wherein the nucleotide at position 150 is substituted with a nucleotide different from the original nucleotide in the nucleotide sequence of SEQ ID NO: 140, and which has at least 70% or more, 80% or more, 85% or more, 90% or more, 95% or more, 96% or more, 97% or more, 98% or more, or 99% or more; and less than 100% homology or identity to the nucleotide sequence of SEQ ID NO: 140, or a nucleotide sequence having the homology or identity in which some sequences are added, deleted, or modified.

In the present disclosure, the nucleotide sequence of SEQ ID NO: 138 may be a promoter sequence of a gene encoding an IclR family transcriptional regulator (CEY17_07095 gene), or a portion of the promoter sequence.

The IclR family transcriptional regulator is a transcriptional regulator mainly involved in carbon metabolism regulation, and functions to regulate the expression of genes associated with the glyoxylate cycle.

In one embodiment, the nucleotide at position 130 in the nucleotide sequence of SEQ ID NO: 138 may be cytosine (C), but is not limited thereto.

In one embodiment, the polynucleotide may be a polynucleotide wherein the nucleotide at position 130 in the nucleotide sequence of SEQ ID NO: 138 is substituted with adenine (A), guanine (G), or thymine (T), and more specifically with thymine (T), but is not limited thereto.

In the present disclosure, the nucleotide sequence of SEQ ID NO: 139 may be a promoter sequence of a gene encoding a tricarboxylate transporter (CEY17_14380 gene), or a portion of the promoter sequence.

The tricarboxylate transporter is a protein responsible for transporting tricarboxylic acids, such as citrate, and is located in the cell membrane, where it functions to import tricarboxylic acids from outside the cell into the cell for use in metabolic pathways.

In one embodiment, the nucleotide at position 71 in the nucleotide sequence of SEQ ID NO: 139 may be guanine (G), but is not limited thereto.

In one embodiment, the polynucleotide may be a polynucleotide wherein the nucleotide at position 71 in the nucleotide sequence of SEQ ID NO: 139 is substituted with adenine (A), cytosine (C), or thymine (T), and more specifically with adenine (A), but is not limited thereto.

In the present disclosure, the nucleotide sequence of SEQ ID NO: 140 may be a promoter sequence of the CEY17_06565 gene, or a portion of the promoter sequence.

In one embodiment, the nucleotide at position 150 in the nucleotide sequence of SEQ ID NO: 140 may be guanine (G), but is not limited thereto.

In one embodiment, the polynucleotide may be a polynucleotide wherein the nucleotide at position 150 in the nucleotide sequence of SEQ ID NO: 140 is substituted with adenine (A), cytosine (C), or thymine (T), and more specifically with adenine (A), but is not limited thereto.

In one embodiment, the polynucleotide may comprise, consist essentially of, or consist of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, or 99.35% or more; and less than 100% homology or identity to the nucleotide sequence of SEQ ID NO: 138. More specifically, the polynucleotide may be a polynucleotide wherein the nucleotide at position 130 of the nucleotide sequence of SEQ ID NO: 138 is substituted with thymine (T), and which comprises, consists essentially of, or consists of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, or 99.35% or more; and less than 100% homology or identity to the nucleotide sequence of SEQ ID NO: 138.

In one embodiment, the polynucleotide may comprise, consist essentially of, or consist of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, 99.4% or more, or 99.5% or more; and less than 100% homology or identity to the nucleotide sequence of SEQ ID NO: 139. More specifically, the polynucleotide may be a polynucleotide wherein the nucleotide at position 71 of the nucleotide sequence of SEQ ID NO: 139 is substituted with adenine (A), and which comprises, consists essentially of, or consists of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, 99.4% or more, or 99.5% or more; and less than 100% homology or identity to the nucleotide sequence of SEQ ID NO: 139.

In one embodiment, the polynucleotide may comprise, consist essentially of, or consist of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, 99.4% or more, or 99.5% or more; and less than 100% homology or identity to the nucleotide sequence of SEQ ID NO: 140. More specifically, the polynucleotide may be a polynucleotide wherein the nucleotide at position 150 of the nucleotide sequence of SEQ ID NO: 140 is substituted with adenine (A), and which comprises, consists essentially of, or consists of a nucleotide sequence having at least 70% or more, 80% or more, 85% or more, 90% or more, 95% or more, 96% or more, 97% or more, 98% or more, 99% or more, 99.1% or more, 99.2% or more, 99.3% or more, 99.4% or more, or 99.5% or more; and less than 100% homology or identity to the nucleotide sequence of SEQ ID NO: 139.

In the present disclosure, the polynucleotide may comprise the nucleotide sequence of SEQ ID NO: 18, SEQ ID NO: 20, or SEQ ID NO: 25. In another embodiment, the polynucleotide of the present disclosure may essentially consist of the nucleotide sequence of SEQ ID NO: 18, SEQ ID NO: 20, or SEQ ID NO: 25. In yet another embodiment, the polynucleotide of the present disclosure may consist of the nucleotide sequence of SEQ ID NO: 18, SEQ ID NO: 20, or SEQ ID NO: 25.

In the present disclosure, the term "polynucleotide" may refer to one in which 2 or more, 5 or more, 10 or more, 13 or more, 20 or more, or 30 or more nucleotide monomers are included, wherein the nucleotide monomers may be covalently linked to form a chain form.

The polynucleotide of the present disclosure may have a promoter activity and may be used as a universal promoter.

In one embodiment, the polynucleotide may have a promoter activity for the expression of a target gene in microorganisms of the genus *Corynebacterium*.

The polynucleotide having promoter activity may be used interchangeably with "variant promoter" in the present disclosure.

The polynucleotide according to one embodiment of the present disclosure may be utilized as a synthetic promoter having strong activity of inducing expression.

In the present disclosure, the term "promoter" may refer to a DNA region that includes a binding site for a polymerase and initiates transcription of a target DNA located at its downstream. The promoter may be located upstream of a transcription start site. The promoter may be operably and/or regulably (enhancing or weakening expression) linked upstream of the target gene. For example, the promoter may be linked in a forward direction on the upstream side of the target gene to enhance (increase) the expression of the gene, or may be linked in a reverse direction on the downstream side of the target gene to weaken (decrease) the expression of the gene. When a promoter is introduced in a reverse direction to downstream of a target gene, for example, downstream of a stop codon, specifically between the stop codon and the top of a transcription terminator, it may weaken the expression of the gene by inducing transcription in the direction opposite to the normal transcription direction of the corresponding gene, thereby causing an RNA polymerase complex to collide with an RNA polymerase complex of the normal direction during the transcription process.

The polymerase, also called RNA polymerase or DNA-dependent RNA polymerase, may refer to an enzyme that synthesizes a primary transcript RNA from DNA. The polymerase may be an RNA polymerase of a prokaryotic cell or an RNA polymerase of a eukaryotic cell (for example, RNA polymerase I, RNA polymerase II, RNA polymerase III, RNA polymerase IV, or RNA polymerase V, etc.). The polynucleotide according to one embodiment may be natural or non-natural, and for example, may be a non-natural one synthesized chemically or recombinantly.

In the present disclosure, the term "variation" refers to a genetically or non-genetically stable phenotypic change, and may be used interchangeably with "mutation" in the present disclosure.

The polynucleotide (variant promoter) of the present disclosure may have changed (increased or decreased) promoter activity compared to a polynucleotide that does not include a variation (a wild-type polynucleotide or a polynucleotide prior to mutation). The polynucleotide may regulate (increase or decrease) the expression of a target gene operably linked thereto, or the expression or activity of a protein encoded by the target gene, and furthermore, may regulate the expression of other genes besides the target gene.

The "target gene" means a gene whose expression is intended to be regulated by the polynucleotide of the present disclosure, and in the case of a gene encoding a protein, may be used interchangeably with 'a gene encoding a target protein'. A protein encoded by the target gene may be expressed as a "target protein", and a gene encoding the "target protein" may be expressed as a "target gene".

The amino acid coding sequence of the target gene may be variously modified within a range that does not change the protein sequence encoded by the target gene, due to the degeneracy of the codon or in consideration of a preferred codon (codon usage frequency) in an organism in which the target gene is to be expressed.

The polynucleotide of the present disclosure, when introduced into a suitable host cell together with a target gene operably linked thereto, may have an activity of increasing the production ability (production amount) of a target substance of the host cell, for example, the production ability (production amount) of an amino acid.

In one embodiment, the polynucleotide may be for increasing amino acid production ability (production amount), and specifically, may be for increasing the production ability (production amount) of L-valine.

In addition, the nucleotide sequence of the polynucleotide may be further modified by conventionally known mutagenesis methods, for example, directed evolution and sitedirected mutagenesis, and the like, to the extent that it maintains a corresponding biological activity (promoter activity) and/or a desired activity (e.g., an activity of increasing production of a target substance in a host cell).

Therefore, the polynucleotide of the present disclosure may consist of a nucleotide sequence having at least 70%, 80%, 85%, 90%, 95%, 96%, 97%, 98%, 99%, 99.5%, 99.7% or 99.9% or more homology or identity to the nucleotide sequence of SEQ ID NO: 18, the nucleotide sequence of SEQ ID NO: 20, or the nucleotide sequence of SEQ ID NO: 25, or a nucleotide sequence having the homology or identity in which some sequences are added, deleted, or modified.

In the present disclosure, the term, 'homology' or 'identity' means the degree of similarity between two given amino acid sequences or nucleotide sequences and may be expressed as a percentage. The terms homology and identity may often be used interchangeably.

The sequence homology or identity of a conserved polynucleotide or polypeptide is determined by a standard alignment algorithm, and default gap penalties established by the program used may be employed together. Substantially, homologous or identical sequences may generally hybridize with the entire sequence or a portion thereof under moderately or highly stringent conditions. It is apparent that hybridization also includes hybridization with a polynucleotide containing a general codon or a codon that takes into account codon degeneracy in the polynucleotide.

Whether any two polynucleotide or polypeptide sequences have homology or identity may be determined, for example, by using a known computer algorithm such as the "FASTA" program using default parameters as in Pearson et al (1988) [Proc.Natl. Acad. Sci. USA 85]: 2444. Alternatively, the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or a later version), may be used for the determination (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073)). For example, homology or identity may be determined using BLAST of the National Center for Biotechnology Information (NCBI) database, or ClustalW.

The homology or identity of a polynucleotide or a polypeptide may be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program may be defined as the value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for end gaps.

In one embodiment, a polynucleotide comprising a specific nucleotide sequence provided in the present disclosure may be interpreted as comprising not only the specific nucleotide sequence or a nucleotide sequence substantially equivalent thereto, but also a polynucleotide fragment comprising a nucleotide sequence complementary to the specific nucleotide sequence. Specifically, the polynucleotide having the complementarity can be identified under the conditions described below: these conditions are specifically described in known literature. For example, conditions under which genes having high complementarity of 60% or more, 70% or more, 80% or more, 85% or more, 90% or more, 91% or more, 92% or more, 93% or more, 94% or more, 95% or more, 96% or more, 97% or more, 98% or more, 98% or more, 99.5% or more, or 99.9% or more, hybridize with each other, and the genes having lower complementarity do not hybridize, or conditions of washing once, specifically two to three times, at a salt concentration and temperature corresponding to the washing conditions of a conventional Southern hybridization, i.e., 60°C, 1x SSC (saline-sodium citrate buffer), and 0.1% (w/v) SDS (Sodium Dodecyl Sulfate); 60°C, 0.1x SSC, and 0.1% SDS; or 68°C, 0.1x SSC, and 0.1% SDS, may be listed, but are not limited thereto. Hybridization requires that two nucleotides have complementary sequences, but some base mismatches may be allowed depending on the stringency of hybridization. The term "complementary" may be used to describe the relationship between nucleotide that can hybridize with each other. For example, in the case of DNA, adenosine is complementary to thymine and cytosine is complementary to guanine. The stringency of hybridization between polynucleotides depends on the length and degree of complementarity of the polynucleotides, which is well known in the relevant art (see Sambrook et al., supra, 9.50-9.51, 11.7-11.8).

Furthermore, the polynucleotide of the present disclosure may be operably linked to a gene encoding a target protein, i.e., a target gene.

In the present disclosure, the term "operably linked (operatively linked)" means that a polynucleotide having promoter activity of the present disclosure is functionally linked to the gene sequence so as to initiate and mediate transcription of the target gene. An operable linkage may be prepared using known gene recombination techniques in the art, and sitespecific DNA cleavage and ligation may be produced using cleavage and ligation enzymes and the like in the art, but are not limited thereto.

When the polynucleotide is operably linked to the target gene, some nucleotides may be added, deleted, and/or modified for the use of the cleavage and ligation enzymes and the like.

In one embodiment, the target gene may be a gene encoding a protein involved in L-valine production according to the present disclosure, but is not limited thereto. The protein involved in the production of L-valine may be a protein involved in at least one process or step of an intracellular production pathway (e.g., biosynthesis, metabolism, bioconversion, etc.), intracellular transport, and/or an extracellular excretion pathway of L-valine, and may be selected from the group consisting of, for example, an enzyme (various synthases, lyases, kinases, carboxylases (e.g., pyruvate carboxylase, etc.), reductases, oxidases, decarboxylases, dehydrogenases, dehydratases, transferases, epimerases, etc.), an intermediate, a transport protein, a membrane protein (channel, etc.), and the like, but is not limited thereto.

In one embodiment, the target gene may be the CEY17_07095 gene, but is not limited thereto. The CEY17_07095 gene may be a gene encoding an IclR family transcriptional regulator.

In one embodiment, the target gene may be the CEY17_14380 gene, but is not limited thereto. The CEY17_14380 gene may be a gene encoding a tricarboxylate transporter.

In one embodiment, the target gene may be the CEY17_06565 gene, but is not limited thereto.

Another aspect of the present disclosure provides an expression cassette comprising a polynucleotide of the present disclosure and a target gene.

The polynucleotide and target gene are as described above.

In the present disclosure, the term, "expression cassette," refers to a unit cassette that comprises a promoter and a target gene operably linked thereto, and is capable of expressing a target gene located downstream of the promoter. Inside or outside such a gene expression cassette, various factors that may aid in the efficient expression of the target gene may be included. The gene expression cassette, in addition to the promoter operably linked to the target gene, may typically include a transcription termination signal, a ribosome binding site, and a translation termination signal, but is not limited thereto.

Another aspect of the present disclosure provides a vector comprising the polynucleotide; the polynucleotide and a target gene operably linked thereto; or the expression cassette of the present disclosure.

The polynucleotide, the target gene, and the expression cassette are as described above. The vector may include a target gene operably linked to the polynucleotide.

In the present disclosure, the term "vector" refers to a DNA construct that contains the nucleotide sequence of the polynucleotide encoding the target protein, operably linked to a suitable regulatory sequence, so as to be able to express the target protein in a suitable host. The regulatory sequence may include a promoter capable of initiating transcription, any operator sequence for regulating transcription, a sequence encoding a suitable mRNA ribosome binding site, and/or a sequence that regulates the termination of transcription and/or translation. After being transformed into a suitable host cell, a vector may be expressed independently of the genome of the host cell or may be integrated into the genome of the host cell.

A vector usable in the present disclosure is not particularly limited as long as it is replicable in a host cell, and may be selected from among all commonly used vectors. Examples of commonly used vectors include plasmids, cosmids, viruses, bacteriophages, and the like, in a natural or recombinant state. For example, as the vector, pWE15, M13, MBL3, MBL4, IXII, ASHII, APII, t10, t11, Charon4A, and Charon21A, and the like may be used as phage vectors or cosmid vectors, and pDZ series, pBR series, pUC series, pBluescriptII series, pGEM series, pTZ series, pCL series, and pET series, and the like may be used as plasmid vectors. Specifically, examples may include, but are not limited to, pDZ, pDC, pACYC177, pACYC184, pCL, pECCG117, pUC19, pBR322, pMW118, pCC1BAC, pDCM2, pDC24 vectors, and the like.

A vector usable in the present disclosure may be an expression vector or an insertion vector for integration into a host cell chromosome. The insertion of the target DNA into the host cell chromosome using an insertion vector may be performed by any method known in the art, for example, by homologous recombination or a CRISPR system, but is not limited thereto. The vector may further include a selection marker for confirming whether the vector has been transformed or further whether the target DNA has been inserted into the chromosome. The selection marker may be selected and used from among genes that confer a selectable phenotype, such as drug resistance, auxotrophy, resistance to cytotoxic agents, or expression of surface proteins. In an environment treated with a selective agent, only cells expressing the selection marker can survive or exhibit a different phenotype, and thus, transformed cells may be selected.

In the present disclosure, the term "transformation" refers to introducing a target polynucleotide into a host cell. A transformed polynucleotide may be inserted into the chromosome of the host cell or may be located extrachromosomally. Furthermore, the polynucleotide may be DNA and/or RNA, and regardless of the form in which it is introduced, as long as it may be introduced into a host cell and function therein. For example, the polynucleotide may be introduced into a host cell in the form of an expression cassette, which is a gene construct including all elements necessary for its own expression, or in the form of a vector including the same.

The transformation method includes any method for introducing the target polynucleotide into a cell, and may be performed by selecting a suitable standard technique as is known in the art, depending on the host cell. For example, examples include electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method, and the like, but it is not limited thereto.

Another aspect of the present disclosure provides a microorganism (host cell) comprising the polynucleotide, the polynucleotide and a target gene operably linked thereto, or the expression cassette of the present disclosure.

The polynucleotide, the target gene, or the expression cassette are as described above.

The polynucleotide, the polynucleotide and the target gene operably linked thereto, or the expression cassette may be introduced into a microorganism by transformation, but is not limited thereto.

In the present disclosure, the term "microorganism" includes both a wild-type microorganism and a microorganism that has been naturally or artificially genetically modified, and also includes a microorganism in which a specific mechanism is attenuated or enhanced due to causes such as the insertion of an exogenous gene or the enhancement or attenuation of the activity of an endogenous gene.

The microorganism may be a microorganism that naturally expresses a target gene or a microorganism that has a target product production ability, or may be a microorganism in which target gene expression ability or target product production ability is conferred to a microorganism that does not naturally express a target gene or to a parent strain that does not have target product production ability, but is not limited thereto. In one embodiment, the microorganism may be a microorganism that naturally produces an amino acid, specifically a microorganism that produces L-valine.

In the present disclosure, the term "target product" refers to a biologically active substance that is intended to be produced or whose production is intended to be regulated (increased or decreased) by using the polynucleotide, the polynucleotide and a target gene operably linked thereto, an expression cassette comprising the polynucleotide and the target gene, a vector comprising the same, and/or a microorganism comprising the same provided in the present disclosure, and is intended to include not only the biologically active substance to be finally produced but also a target protein that may be produced by the microorganism. For example, it may refer to the target protein itself encoded by the target gene, and/or any biologically active substance produced through the involvement of the target protein. The biologically active substance refers to any substance that is produced or derived from an organism (e.g., a cell) or has a predetermined function in vivo or within a cell, and may be, for example, an amino acid (glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, O-acetyl homoserine, etc.), a nucleic acid, a vitamin (vitamin A, B (B1, B2, B3, B5, B6, B7, B9, B12, etc.), C, D, E, K, etc.), a protein (the target protein or a protein other than the target protein, for example, a hormone, a growth factor, a cytokine, an immunoglobulin (antibody), an antigen protein, a receptor, a ligand, a functional fragment thereof (a fragment retaining a desired function), a fusion protein in which two or more are fused, etc.), a sugar (e.g., a monosaccharide, a disaccharide, a polysaccharide, a sugar alcohol, etc.), a fatty acid (myristoleic acid, palmitoleic acid, sapienic acid, oleic acid, elaidic acid, vaccenic acid, linoleic acid, linoelaidic acid, arachidonic acid, eicosapentaenoic acid (EPA), erucic acid, docosahexaenoic acid (DHA), etc.), an organic acid (lactic acid, citric acid, oxalic acid, uric acid, butyric acid, stearic acid, propionic acid, etc.), and the like, but is not limited thereto. In addition, if it is a substance produced through the involvement of the target protein, metabolites thereof (polyhydroxyalkanoate (PHA), etc.), precursors thereof, derivatives thereof that maintain their biological activity, and the like may also be included in the target product, in addition to the substances described above.

In one embodiment, the microorganism comprising the polynucleotide and a target gene operably linked thereto of the present disclosure may be a microorganism with an increased production ability of an amino acid as a target product, and specifically may be a microorganism with an increased production ability of L-valine. The microorganism with the increased production ability of L-valine may be a microorganism with an increased production ability of L-valine compared to a microorganism that does not comprise the polynucleotide of the present disclosure, for example, a microorganism in which the expression of the CEY17_07095 gene, the CEY17_14380 gene, or the CEY17_06565 gene is regulated by a polynucleotide prior to introduction of a mutation, but is not limited thereto. The polynucleotide before the introduction of the mutation may be a wild-type polynucleotide, and specifically may consist of the nucleotide sequence of SEQ ID NO: 138, SEQ ID NO: 139, or SEQ ID NO: 140.

In the present disclosure, an "unmodified microorganism" does not exclude a strain including a naturally occurring mutation, and may refer to a wild-type strain or a naturally occurring strain itself, or a strain before its properties are changed by genetic variation due to natural or artificial factors. For example, the unmodified microorganism may refer to a strain in which a variant polynucleotide has not been introduced into the promoter region of the CEY17_07095 gene, the CEY17_14380 gene, and/or the CEY17_06565 gene described in the present disclosure, or a strain before such introduction. The "unmodified microorganism" may be used interchangeably with "pre-modification strain", "pre-modification microorganism", "non-mutant strain", "unmodified strain", "non-mutant microorganism", or "reference microorganism".

The microorganism of the present disclosure may comprise, without limitation, any microorganism into which the polynucleotide of the present disclosure may be introduced and function as a promoter.

In one embodiment, the microorganism may be a microorganism of the genus *Corynebacterium* (*Corynebacterium* sp.), a microorganism of the genus *Escherichia*(*Escherichia* sp.), or a microorganism of the genus *Bacillus* (*Bacillus* sp.), but is not limited thereto.

Specifically, the microorganism may be a microorganism of the genus *Corynebacterium*, and more specifically, may include *Corynebacterium glutamicum, Corynebacterium stationis, Corynebacterium thermoaminogenes, Corynebacterium glutamicum, Brevibacterium flavum, Brevibacterium lactofermentum*, and strains prepared therefrom, but is not limited thereto. Specifically, the microorganism of the genus *Corynebacterium* may be *Corynebacterium glutamicum*.

In one embodiment, the microorganism may be *Corynebacterium glutamicum* in which a mutation has been introduced into the promoter of the CEY17_07095 gene, the CEY17_14380 gene, or the CEY17_06565 gene, but is not limited thereto.

In one embodiment, the microorganism of the present disclosure with increased L-valine production ability (production amount) may have an L-valine production ability (production amount) increased by about 0.5% or more, about 1.0% or more, about 2.0% or more, about 3.0% or more, about 3.5% or more, about 3.75% or more, about 4.0% or more, about 4.25% or more, about 4.3% or more, about 4.5% or more, about 4.75% or more, about 4.8% or more (the upper limit is not particularly limited, and may be, for example, about 200% or less, about 100% or less, about 50% or less, about 25% or less, or about 10% or less) compared to a parent strain before mutation or an unmodified microorganism, but is not limited thereto. In another embodiment, the microorganism of the present disclosure with increased L-valine production ability (production amount) may have an L-valine production ability (production amount) increased by about 1.005-fold or more, about 1.01-fold or more, about 1.02-fold or more, about 1.03-fold or more, about 1.035-fold or more, about 1.0375-fold or more, about 1.04-fold or more, about 1.0425-fold or more, about 1.043-fold or more, about 1.045-fold or more, about 1.0475-fold or more, or about 1.048-fold or more (the upper limit is not particularly limited, and may be, for example, about 5-fold or less, about 2-fold or less, about 1.5-fold or less, about 1.2-fold or less, or about 1.1-fold or less) compared to a parent strain before mutation or an unmodified microorganism, but is not limited thereto.

The term "about" refers to a range that encompasses all of ±0.5, ±0.4, ±0.3, ±0.2, ±0.1, ±0.05, ±0.04, ±0.03, ±0.02, ±0.01, etc., and includes all numerical values in a range equivalent or similar to the numerical value that follows the term about, but is not limited thereto.

Another aspect of the present disclosure provides a composition for producing L-valine, comprising at least one selected from the group consisting of the polynucleotide, the polynucleotide and a target gene operably linked thereto, the expression cassette, the vector, and the microorganism of the present disclosure. The polynucleotide, the target gene, the expression cassette, the vector, the microorganism, and the like are as described above.

In one embodiment, the composition for producing L-valine may further comprise any suitable excipient conventionally used in a composition for producing L-valine, and such an excipient may be, for example, a preservative, a wetting agent, a dispersing agent, a suspending agent, a buffer, a stabilizer, or an isotonic agent, and the like, but is not limited thereto.

Another aspect of the present disclosure provides a use of at least one selected from the group consisting of the polynucleotide, the polynucleotide and the target gene operably linked thereto, the expression cassette, the vector, and the microorganism of the present disclosure for producing L-valine. The polynucleotide, the target gene, the expression cassette, the vector, the microorganism, and the like are as described above.

Another aspect of the present disclosure provides a use of at least one selected from the group consisting of the polynucleotide, the polynucleotide and a target gene operably linked thereto, the expression cassette, the vector, and the microorganism of the present disclosure for preparing a composition for producing L-valine. The polynucleotide, the target gene, the expression cassette, the vector, the microorganism, and the like are as described above.

Another aspect of the present disclosure provides a method for producing a target product, the method comprising a step of culturing in a medium a microorganism comprising the polynucleotide, the polynucleotide and a target gene operably linked thereto, the expression cassette or the vector of the present disclosure.

The polynucleotide, the target gene, the expression cassette, the vector, the microorganism, and the target product, and the like, are as described above.

In one embodiment, the target product may be an L-amino acid. Specifically, the target product may be L-valine.

The method may further comprise a step of recovering the target product from the cultured microorganism, the culture, or both, after the step of culturing.

The term "culturing" in the present disclosure means growing a microorganism under appropriately and artificially controlled environmental conditions. The culturing process of the present disclosure may be performed according to a suitable medium and culture conditions known in the art. Such a culturing process may be easily adjusted and used by a person skilled in the art according to a selected strain. Specifically, the culturing may be a batch, continuous, and/or fed-batch culture, but is not limited thereto. These various methods are disclosed in, for example, "Biochemical Engineering" (James M. Lee, Prentice-Hall International Editions, pp138-176, 1991), and the like.

In the present disclosure, the term "medium" means a substance prepared by mixing nutrients required for culturing the microorganism of the present disclosure as main components, and supplies water, which is essential for survival and growth, as well as nutrients and growth factors. Specifically, as the medium and other culture conditions used for culturing the microorganism of the present disclosure, any medium used for culturing conventional microorganisms may be used without particular limitation, but the microorganism of the present disclosure may be cultured in a conventional medium containing a suitable carbon source, nitrogen source, phosphorus source, inorganic compound, amino acid and/or vitamin, while controlling temperature, pH, etc., under aerobic conditions. Specifically, a culture medium for a microorganism may be found in literature such as ["Manual of Methods for General Bacteriology" by the American Society for Bacteriology (Washington D.C., USA, 1981)].

The medium used for culturing must meet the requirements of a specific strain in an appropriate manner. For example, culturing may be performed in a conventional medium containing a suitable carbon source, nitrogen source, amino acid, vitamin, etc., while controlling temperature, pH, etc., under aerobic conditions. At this time, the carbon source includes carbohydrates such as glucose, fructose, and sucrose, and amino acids such as glutamic acid and cysteine. Specifically, natural organic nutrient sources such as starch hydrolysates and molasses may be used, and preferably carbohydrates such as glucose, fructose, sterilized pre-treated molasses (i.e., molasses converted to reducing sugars), and other appropriate amounts of carbon sources may be used in various ways without limitation, but are not limited thereto. As the nitrogen source, inorganic nitrogen sources such as ammonia; and amino acids such as glutamic acid and cysteine, and peptone, meat extract, yeast extract, etc., may be used as organic nitrogen sources. These nitrogen sources may be used alone or in combination, but are not limited thereto. In the medium, phosphoric acid, potassium dihydrogen phosphate or dipotassium hydrogen phosphate, or a corresponding sodium-containing salt may be used as a phosphorus source, but is not limited thereto. As the inorganic compounds, magnesium sulfate, iron sulfate, manganese sulfate, and calcium chloride, etc., may be used, and in addition, amino acids, vitamins, and appropriate precursors, etc., may be included. These media or precursors may be added to the culture in a batch or continuous manner, but are not limited thereto.

During culturing, the pH of the culture may be adjusted by adding compounds such as potassium hydroxide, ammonia, and phosphoric acid to the culture in an appropriate manner. Furthermore, during culturing, an antifoaming agent such as a fatty acid polyglycol ester may be used to suppress foam generation. Furthermore, to maintain the aerobic state of the culture, oxygen or an oxygen-containing gas may be injected into the culture. The temperature of the culture is 27°C to 37°C, and specifically 30°C to 33°C. The culturing period may be continued until the desired production amount of the useful substance is obtained, and is specifically 20 to 160 hours.

In the present disclosure, the term "culture" means a substance comprising a medium in which a microorganism is growing or has completed growth under appropriately and artificially controlled environmental conditions. In a narrow sense, the culture does not include the grown microorganism, but it may be included in a broad sense. The "culture" may include various target substances discharged by the microorganism into the medium during its growth, along with the medium components formulated for the microorganism culture.

In the culturing of the present disclosure, the culture temperature may be maintained at 20°C to 45°C, specifically 25°C to 40°C, 25°C to 40°C, 25°C to 37°C, 25°C to 35°C, 27°C to 40°C, 27°C to 37°C, 27°C to 35°C, 30°C to 40°C, 30°C to 37°C, or 30°C to 35°C, and the culturing may be performed for about 10 to 160 hours, 20 to 100 hours, 30 to 90 hours, or 50 to 70 hours, but is not limited thereto.

The target product produced by the culturing of the present disclosure may be secreted into the medium or may be retained within the cells.

The step of recovering the target product may be to collect the target product using a suitable method known in the art, according to the culturing method, for example, a batch, continuous, or fed-batch culturing method. For example, centrifugation, filtration, treatment with a crystallizing protein precipitating agent (salting out), extraction, ultrasonic disruption, ultrafiltration, dialysis, various types of chromatography such as molecular sieve chromatography (gel filtration), adsorption chromatography, ion-exchange chromatography, and affinity chromatography, HPLC, or a combination of these methods may be used, but is not limited thereto, and the desired target product may be recovered from the medium or the microorganism using a suitable method known in the art.

Furthermore, the method for producing a target product of the present disclosure may additionally comprise a purification step. The purification may be performed using a suitable method known in the art. In one embodiment, when the method for producing a target product of the present disclosure comprises both a recovery step and a purification step, the recovery step and the purification step may be performed separately (or continuously) regardless of the order, or may be performed simultaneously or integrated into a single step, but is not limited thereto.

Another aspect of the present disclosure provides a method for increasing the production ability of a target product, the method comprising a step of culturing in a medium a microorganism comprising the polynucleotide, the polynucleotide and a target gene, or an expression cassette comprising the polynucleotide and a target gene of the present disclosure.

The polynucleotide, the target gene, the expression cassette, the microorganism, and the target product, and the like, are as described above.

Another aspect of the present disclosure provides a method for preparing a microorganism with increased production ability of a target product, the method comprising a step of introducing into a microorganism the polynucleotide, the polynucleotide and a target gene, or an expression cassette comprising the polynucleotide and a target gene of the present disclosure.

The polynucleotide, the target gene, the expression cassette, the microorganism, and the target product, and the like, are as described above.

According to another aspect of the present disclosure, the present disclosure provides a composition, a method, a product, a process, or a use characterized by one or more elements disclosed in the present disclosure.

### [ADVANTAGEOUS EFFECTS]

The present invention relates to a novel polynucleotide having a promoter activity and a method for producing L-valine using the same, and as the L-valine production ability is significantly increased in a microorganism introduced with the novel polynucleotide of the present invention, the novel polynucleotide can be usefully utilized for efficiently producing L-valine.

### [MODE FOR CARRYING OUT THE INVENTION]

Hereinafter, the present application will be described in more detail through examples. These examples are intended solely to illustrate the present application more specifically, and it will be apparent to those skilled in the art that the scope of the present application is not limited by these examples, in accordance with the gist of the present application.

### EXAMPLE

(Throughout this specification, the term "%" used to indicate the concentration of a specific substance, unless otherwise specified, refers to (weight/weight) % for solid/solid, (weight/volume) % for solid/liquid, and (volume/volume) % for liquid/liquid.)

### Example 1. Selection of mutant strains with increased L-valine production ability using an artificial mutagenesis method

### Example 1-1. Random mutagenesis via UV irradiation

In order to select a mutant strain with enhanced L-valine production, CJ7V, a valine-producing strain (Biotechnology and Bioprocess Engineering, June 2014, Volume 19, Issue 3, pp. 456-467; Korean Patent No. 10-2263091; US 2023-0086419 A1), was spread onto a nutrient medium containing agar and cultured at 30°C for 16 hours. Hundreds of colonies thus obtained were exposed to ultraviolet (UV) irradiation at room temperature to induce random mutations in the genome of the strain. The composition of the nutrient medium is as follows.

### <Nutrient Medium (pH 7.2)>

Glucose 10 g, meat extract 5 g, polypeptone 10 g, sodium chloride 2.5 g, yeast extract 5 g, agar 20 g, urea 2 g (based on 1 L of distilled water)

### Example 1-2. Selection of strains with increased L-valine production ability

In order to select a mutant strain with increased valine production ability compared to the parent strain CJ7V, the CJ7V strain and mutant strains in which random mutations were induced in Example 1-1 were cultured as follows.

Each of the aforementioned strains was inoculated into a 96-Deep Well Plate-Dome (Bioneer) containing 400 µl of seed medium, and cultured in a plate shaking incubator (TAITEC) at 32°C and 1200 rpm for about 48 hours. The composition of the seed medium is as follows.

### <Seed Medium (pH 7.0)>

Glucose 50 g, peptone 10 g, yeast extract 10 g, urea 4 g, sodium chloride 2.5 g (based on 1 L of distilled water)

The L-valine concentration of approximately 3,000 cultured strains was individually measured using near-infrared spectroscopy (NIR), and the top six strains showing increased L-valine production ability compared to CJ7V were selected.

### Example 1-3. Analysis of L-valine production ability of selected mutant strains

In order to finally select strains having increased L-valine production ability from the six strains selected in Example 1-2, a reproducibility test was conducted in flasks using the following medium.

First, each strain was subcultured in a nutrient medium, and then inoculated into a 250 mL corner-baffle flask containing 25 mL of a production medium, followed by shake culture at 30°C and 200 rpm for 72 hours. The compositions of the nutrient medium and the production medium are as follows.

### <Nutrient Medium (pH 7.2)>

Glucose 10 g, Beef extract 5 g, polypeptone 10 g, sodium chloride 2.5 g, yeast extract 5 g, agar 20 g, urea 2 g (based on 1 L of distilled water)

### <Production Medium (pH 7.0)>

Glucose 100 g, ammonium sulfate 40 g, soybean protein 2.5 g, corn steep solids 5 g, urea 3 g, dipotassium phosphate 1 g, magnesium sulfate heptahydrate 0.5 g, biotin 100 ug, thiamine-HCl 1 mg, calcium pantothenate 2 mg, nicotinamide 3 mg, calcium carbonate 30 g

### (based on 1 L of distilled water)

After completion of the culture, the L-valine concentration in the culture broth was analyzed using high-performance liquid chromatography (HPLC), and the L-valine production concentrations of each mutant strain are shown in Table 1 below.

**[Table 1]**

| Strain name | L-valine (g/L) |
|---|---|
| CJ7V | 2.2 |
| CJ7V_mt1 | 2.21 |
| **CJ7V_mt2** | **2.31** |
| CJ7V_mt3 | 2.24 |
| CJ7V_mt4 | 2.19 |
| CJ7V_mt5 | 2.22 |
| CJ7V_mt6 | 2.17 |

Among the selected mutant strains, the mutant strain CJ7V_mt2, which exhibited the greatest increase in L-valine production amount compared to CJ7V, was finally selected.

### Example 2. Mutation identification using whole-genome sequencing (WGS)

Whole-genome sequencing (WGS) was performed on the CJ7V_mt2 strain selected in Example 1, and the obtained sequence was analyzed and compared with that of the parent strain CJ7V to identify mutations occurring in the nucleotide sequence of the strain. Mutations in coding DNA sequence (CDS) regions, promoter regions, rRNA, and tRNA were identified, and the types and sequences of the respective mutations are shown in Tables 2 to 4 below.

**[Table 2]**

| Mutations in CDS regions | | | |
|---|---|---|---|
| Gene name | Expressed protein (regulatory protein) | Variant amino acid sequence | Variant nucleotide sequence |
| CEY17-06240 | NAD(P)/FAD-dependent oxidoreductase | SEQ ID NO: 1 | SEQ ID NO: 2 |
| CEY17_16000 | methylhydantoinase | SEQ ID NO: 3 | SEQ ID NO: 4 |
| CEY17_05275 | AAA family ATPase | SEQ ID NO: 5 | SEQ ID NO: 6 |
| CEY17_06935 | DUF262 domain-containing protein | SEQ ID NO: 7 | SEQ ID NO: 8 |
| CEY17_04835 | 16S rRNA (cytidine(1402)-2'-O)-methyltransferase | SEQ ID NO: 9 | SEQ ID NO: 10 |
| CEY17_06635 | co-chaperone YbbN | SEQ ID NO: 11 | SEQ ID NO: 12 |

**[Table 3]**

| Mutations in promoter regions | | |
|---|---|---|
| Name | Protein regulated by the promoter | Variant nucleotide sequence |
| PCEY17_16100 | 50S ribosomal protein L34 | SEQ ID NO: 13 |
| PCEY17_03290 | transporter | SEQ ID NO: 14 |
| PCEY17_01650 | WhiB family transcriptional regulator | SEQ ID NO: 15 |
| PCEY17_01655 | DUF4177 domain-containing protein | SEQ ID NO: 16 |
| PCEY17_02820 | 30S ribosomal protein S12 | SEQ ID NO: 17 |
| PCEY17_07095 | IclR family transcriptional regulator | SEQ ID NO: 18 |
| PCEY17_15935 | nucleotide pyrophosphohydrolase | SEQ ID NO: 19 |
| PCEY17_14380 | tricarboxylic transporter | SEQ ID NO: 20 |
| PCEY17_01395 | hypothetical protein | SEQ ID NO: 21 |
| PCEY17_00355 | stress protein | SEQ ID NO: 22 |
| PCEY17_10495 | hypothetical protein | SEQ ID NO: 23 |
| PCEY17_15010 | GIsB/YeaQ/YmgE family stress response membrane protein | SEQ ID NO: 24 |
| PCEY17_06565 | hypothetical protein | SEQ ID NO: 25 |
| PCEY17_05270 | hypothetical protein | SEQ ID NO: 26 |
| PCEY17_06260 | peptide synthetase | SEQ ID NO: 27 |

**[Table 4]**

| Mutations in RNA regions (rRNA and tRNA) | | | |
|---|---|---|---|
| Category | Name | RNA | Variant nucleotide sequence |
| rRNA | CEY17_12995 | 23S ribosomal RNA | SEQ ID NO: 28 |
| rRNA | CEY17_15045 | 23S ribosomal RNA | SEQ ID NO: 29 |
| rRNA | CEY17_00510 | 16S ribosomal RNA | SEQ ID NO: 30 |
| tRNA | CEY17_12940 | tRNA-Ala | SEQ ID NO: 31 |
| tRNA | CEY17_00330 | tRNA-Leu | SEQ ID NO: 32 |

In the following Examples, each of the mutations listed in Tables 2 to 4 was evaluated to determine whether it affects the L-valine production ability of a microorganism of the genus *Corynebacterium,* thereby identifying effective factors influencing L-valine production ability.

### Example 3. Construction of an L-valine-producing strain into which a variant polynucleotide is introduced

### Example 3-1. Construction of a recombinant vector for introducing a variant polynucleotide

In order to introduce each of the mutations shown in Tables 2 to 4 into the chromosome of CJ7V, vectors containing the respective target mutations were constructed.

Specifically, genomic DNA of the CJ7V_mt2 strain was extracted using a G-spin Total DNA Extraction Mini Kit (Intron, Cat. No. 17045) according to the protocol provided in the kit, and PCR was performed using the genomic DNA as a template. The polymerase used was Solg^{™} Pfu-X DNA polymerase, and the PCR conditions were as follows: denaturation at 95°C for 4 minutes; followed by 27 cycles of denaturation at 95°C for 30 seconds, annealing at 60°C for 30 seconds, and extension at 72°C for 50 seconds; and a final extension at 72°C for 5 minutes. The sequences of the primer pairs used in the experiment are shown in Table 5 below.

**[Table 5]**

| SEQ ID NO: | Name | Sequence (5'->3') |
|---|---|---|
| 33 | CEY17-06240*_F | tgaattcgagctcggtacccCTCAAGTATGTACCGAAACC |
| 34 | CEY17-06240*_R | gtcgactctagaggatccccTCCGGGTACAGGTTGTTTGG |
| 35 | CEY17_16000*_F | tgaattcgagctcggtacccAGACCGACTATGTTCAGAAA |
| 36 | CEY17_16000*_R | gtcgactctagaggatccccGCGTGCGACTATCTGTTCAT |
| 37 | CEY17_05275*_F | tgaattcgagctcggtacccTCCATGTGGTCGACCCCGGC |
| 38 | CEY17_05275*_R | gtcgactctagaggatccccCTCGTAGACAGCTCGCCCAG |
| 39 | CEY17_06935*_F | tgaattcgagctcggtacccTCAGTCGACGAAACCGGCAA |
| 40 | CEY17_06935*_R | gtcgactctagaggatccccGGCCATTTCTTCTGCATCAA |
| 41 | CEY17_04835*_F | tgaattcgagctcggtacccTGGCGCGCATTTGTGGGAAG |
| 42 | CEY17_04835*_R | gtcgactctagaggatccccGGTGATCTCGCCACGCACCC |
| 43 | CEY17_06635*_F | tgaattcgagctcggtacccTATTCGTCCGGGACACACTG |
| 44 | CEY17_06635*_R | gtcgactctagaggatccccCGATATTGTGCCCGAATACT |
| 45 | PCEY17_16100*_F | tgaattcgagctcggtacccGCGACGCCCTTTCCTCATCA |
| 46 | PCEY17_16100*_R | gtcgactctagaggatccccAGTTCAATCCACATGTCCAA |
| 47 | PCEY17_03290*_F | tgaattcgagctcggtacccATGGGGCGGTGGCTTGGTGG |
| 48 | PCEY17_03290*_R | gtcgactctagaggatccccCAGTGACTTAATAAGCACCC |
| 49 | PCEY17_01650*_F | tgaattcgagctcggtacccAGAATTCTTCTACAAGAAAA |
| 50 | PCEY17_01650*_R | gtcgactctagaggatccccAACCTACTCACATTGGATAA |
| 51 | PCEY17_01655*_F | tgaattcgagctcggtacccGTCATGACGTCTGTGATTCC |
| 52 | PCEY17_01655*_R | gtcgactctagaggatccccGTAAGCAACGAGGTTCTCAG |
| 53 | PCEY17_02820*_F | tgaattcgagctcggtacccTTCCATGCTTTGGAGGAGCG |
| 54 | PCEY17_02820*_R | gtcgactctagaggatccccGTGTTCCTGCAGGTTGTGGC |
| 55 | PCEY17_07095*_F | tgaattcgagctcggtacccGCGATAAAGCTGAACGGATT |
| 56 | PCEY17_07095*_R | gtcgactctagaggatccccGGTCGATGTAGAGGAGGTCG |
| 57 | PCEY17_15935*_F | tgaattcgagctcggtacccCTGCGCAAATCTACCTGTCG |
| 58 | PCEY17_15935*_R | gtcgactctagaggatccccTGAAATTGTCCCCAGTCGCG |
| 59 | PCEY17_14380*_F | tgaattcgagctcggtacccCCTGAATATTGTTCACGATC |
| 60 | PCEY17_14380*_R | gtcgactctagaggatccccCAGAAATTTGGGAGTTTAGT |
| 61 | PCEY17_01395*_F | tgaattcgagctcggtacccAAGTGCCACACCTGCTCCAC |
| 62 | PCEY17_01395*_R | gtcgactctagaggatccccAGTACAGCCCCAGAAGTAGC |
| 63 | PCEY17_00355*_F | tgaattcgagctcggtacccGGCAGCTTTCTTGGGACTGC |
| 64 | PCEY17_00355*_R | gtcgactctagaggatccccGACCGATACTGGGGCGATGC |
| 65 | PCEY17_10495*_F | tgaattcgagctcggtacccTGGGGCATAAATTGCGATAC |
| 66 | PCEY17_10495*_R | gtcgactctagaggatccccGCTGCAAATCGCCAGTGCCT |
| 67 | PCEY17_15010*_F | tgaattcgagctcggtacccCTATTCCAATTATTCATTGG |
| 68 | PCEY17_15010*_R | gtcgactctagaggatccccAACAGCAGTGGGATGAAGTA |
| 69 | PCEY17_06565*_F | tgaattcgagctcggtacccTCTTAACGGCTCTCAGCGTC |
| 70 | PCEY17_06565*_R | gtcgactctagaggatccccGCTGCCTTGAGGGGTTTCTC |
| 71 | PCEY17_05270*_F | tgaattcgagctcggtacccAGCCTCCATCACGATCGCAC |
| 72 | PCEY17_05270*_R | gtcgactctagaggatccccGCCAATCTGTGCGAAGAAGA |
| 73 | PCEY17_06260*_F | tgaattcgagctcggtacccACCCATGGAGGGGCCGACGC |
| 74 | PCEY17_06260*_R | gtcgactctagaggatccccCATGATCAATCCATTTCCCC |
| 75 | CEY17_12995*_F | tgaattcgagctcggtacccAGTATTCTCTACCTGACTAC |
| 76 | CEY17_12995*_R | gtcgactctagaggatccccGCGTTGATCCGAGGATGTCC |
| 77 | CEY17_15045*_F | tgaattcgagctcggtacccACACGCTCCCAACGAAAAGT |
| 78 | CEY17_15045*_R | gtcgactctagaggatccccGCGAAGACAGCCAGGAGGTTG |
| 79 | CEY17_00510*_F | tgaattcgagctcggtacccTTGTTTTTTGTGGAGAGTTTG |
| 80 | CEY17_00510*_R | gtcgactctagaggatccccTCACAGTATTGACTGTGTTG |
| 81 | CEY17_12940*_F | tgaattcgagctcggtacccATCGGACTCATCACCGCTGA |
| 82 | CEY17_12940*_R | gtcgactctagaggatccccCGCAACGGCGACAACATCCA |
| 83 | CEY17_00330*_F | tgaattcgagctcggtacccGTTCCATAAGTGTCTTCTCC |
| 84 | CEY17_00330*_R | gtcgactctagaggatccccTTCTTCATATTTTCCCCTCG |

The mutation-introduced fragments obtained as described above and the pDC24 vector (SEQ ID NO: 137) treated with the restriction enzyme smaI were cloned using a Gibson assembly method (DG Gibson et al., Nature Methods, Vol. 6, No. 5, May 2009; NEBuilder HiFi DNA Assembly Master Mix) to obtain recombinant plasmids. The vectors comprising the respective mutation-introduced fragments were designated as pDC24-CEY17_06240*; pDC24-CEY17_16000*; pDC24-CEY17_05275*; pDC24-CEY17_06935*; pDC24-CEY17_004835*; pDC24-CEY17_06635*; pDC24-Pn*_CEY17_16100; pDC24-Pn*_CEY17_03290; pDC24-Pn*_CEY1701650; pDC24-Pn*_CEY17_01655; pDC24-Pn*_CEY17_02820; pDC24-Pn*_CEY17_07095; pDC24-Pn*_CEY17_15935; pDC24-Pn*_CEY17_14380; pDC24-Pn*_CEY17_01395; pDC24-Pn*_CEY17_00355; pDC24-Pn*_CEY17_10495; pDC24-Pn*_CEY17_15010; pDC24-Pn*_CEY17_06565; pDC24-Pn*_CEY17_05270; pDC24-Pn*_CEY17_06260; pDC24-CEY17_12995*; pDC24-CEY17_15045*; pDC24-CEY17_00510*; pDC24-CEY17_12940*; and pDC24-CEY17_00300*.

### Example 3-2. Construction of an L-valine-producing strain into which a variant polynucleotide is introduced

The vectors constructed in Example 3-1 were introduced into the L-valine-producing strain CJ7V by electroporation (Appl. Microbiol. Biotechnol. (1999) 52:541-545), and strains in which the vector was integrated into the chromosome by homologous recombination were selected using a kanamycin-containing medium. Thereafter, transformants in which secondary recombination had occurred were analyzed by PCR using the primer pairs shown in Table 6 to confirm the introduction of the variant sequences, and were designated as CJ7V△CEY17_06240::CEY17_06240*; CJ7V△CEY17_16000::CEY17_16000*; CJ7V△CEY17_05275::CEY17_05275*; CJ7V△CEY17_06935::CEY17_06935*; CJ7V△CEY17_04835::CEY17_04835*; CJ7V△CEY17_06635::CEY17_06635*; CJ7V△Pn_CEY17_16100::Pn*_CEY17_16100; CJ7V△Pn_CEY17_03290::Pn*_CEY17_03290; CJ7V△Pn_CEY17_01650::Pn*_CEY17_01650; CJ7V△Pn_CEY17_01655::Pn*_CEY17_01655; CJ7V△Pn_CEY17_02820::Pn*_CEY17_02820; CJ7V△Pn_CEY17_07095::Pn*_CEY17_07095; CJ7V△Pn_CEY17_15935::Pn*_CEY17_15935; CJ7V△Pn_CEY17_14380::Pn*_CEY17_14380; CJ7V△Pn_CEY17_01395::Pn*_CEY17_01395; CJ7V△Pn_CEY17_00355::Pn*_CEY17_00355; CJ7V△Pn_Pn_CEY17_10495::Pn*_CEY17_10495; CJ7V△Pn_CEY17_15010::Pn*_CEY17_15010; CJ7V△Pn_CEY17_06565::Pn*_CEY17_06565; CJ7V△Pn_CEY17_05270::Pn*_CEY17_05270; CJ7V△Pn_CEY17_06260::Pn*_CEY17_06260; CJ7V△CEY17_12995::CEY17_12995*; CJ7V△CEY17_15045::CEY17_15045*; CJ7V△CEY17_00510::CEY17_00510*; CJ7V△CEY17_12940::CEY17_12940*; and CJ7V△CEY17_00300::CEY17_00300*.

**[Table 6]**

| SEQ ID NO: | Name | Sequence (5'->3') |
|---|---|---|
| 85 | CEY17-06240*_CF | TATAGGAGGCATTCATGG |
| 86 | CEY17-06240*_CR | CAATCTGATCATTCTTAG |
| 87 | CEY17_16000*_CF | GTGTCTTGCTCTTCTGTTG |
| 88 | CEY17_16000*_CR | TAGGAGTCATGCCGCCTG |
| 89 | CEY17_05275*_CF | CGCTGGTGATGTGGTCCTG |
| 90 | CEY17_05275*_CR | TCCAATGAGCTGGCGGAA |
| 91 | CEY17_06935*_CF | GCGCGCTGTCCGGTGCCC |
| 92 | CEY17_06935*_CR | ACTCCGGTGTCTTGGGTT |
| 93 | CEY17_04835*_CF | TTCATCAAAAACTGGGGT |
| 94 | CEY17_04835*_CR | TTTATGTTCACGGGAGCA |
| 95 | CEY17_06635*_CF | GTTGCTTTTTCTGTAGCC |
| 96 | CEY17_06635*_CR | AGGGCCTTTTTGGCGCTG |
| 97 | PCEY17_16100*_CF | GGAGAGTAGCTCTGGTGA |
| 98 | PCEY17_16100*_CR | TCCACAATGTTGTCCACA |
| 99 | PCEY17_03290*_CF | GGAAAAACCACATTTACTC |
| 100 | PCEY17_03290*_CR | CTTTGAGCAGTTTCGCTC |
| 101 | PCEY17_01650*_CF | ACTCCAACAATGTCAGCC |
| 102 | PCEY17_01650*_CR | GCATAAACATGCTGGCAG |
| 103 | PCEY17_01655*_CF | CCAAAACTGAATTTAATG |
| 104 | PCEY17_01655*_CR | GCATCCTCAGCGGAAACC |
| 105 | PCEY17_02820*_CF | CGCCCATAAACGAGACCG |
| 106 | PCEY17_02820*_CR | GAGCTGGGTAACGAGCTC |
| 107 | PCEY17_07095*_CF | CGCTCGCCCACGGACTCC |
| 108 | PCEY17_07095*_CR | TGCAGGCGAACACCGAAC |
| 109 | PCEY17_15935*_CF | CTAAATCAATGCGCACAC |
| 110 | PCEY17_15935*_CR | GCCTGCTCGACGGGGTAC |
| 111 | PCEY17_14380*_CF | TGCCTTCCAACCTTCAAT |
| 112 | PCEY17_14380*_CR | AGCACACAAGGGAAAAGA |
| 113 | PCEY17_01395*_CF | CGGCTGATTGTTGTTGTT |
| 114 | PCEY17_01395*_CR | GGGGATTCTCTTTGGTGG |
| 115 | PCEY17_00355*_CF | AAGCGATACCCATGATGA |
| 116 | PCEY17_00355*_CR | GGCCCAAAACTACAATGA |
| 117 | PCEY17_10495*_CF | TTCTGCTTTGTCGGTTGT |
| 118 | PCEY17_10495*_CR | GGAAAGATCGGTGTAGGT |
| 119 | PCEY17_15010*_CF | GAAGATCAAACCGCCACC |
| 120 | PCEY17_15010*_CR | TGCAAAGTGTCCAGTCGT |
| 121 | PCEY17_06565*_CF | CCGTGCCAGTGATGAAAA |
| 122 | PCEY17_06565*_CR | ATTGCGAACCAACCATTAG |
| 123 | PCEY17_05270*_CF | CTTCGGATTCCCACAAAGTC |
| 124 | PCEY17_05270*_CR | AACATCACGAGACCACGGCG |
| 125 | PCEY17_06260*_CF | CTGTAAGCCCAGGAGAGAA |
| 126 | PCEY17_06260*_CR | TGATGAATCTGCTCCGGT |
| 127 | CEY17_12995*_CF | GCACCGGGCAGGCGTCAGTCC |
| 128 | CEY17_12995*_CR | GTCGGTGGTGTGTTGTGTGA |
| 129 | CEY17_15045*_CF | CTCTTAACCTTCCAGCACCG |
| 130 | CEY17_15045*_CR | CTTAGGTTGAAAACTGAGGG |
| 131 | CEY17_00510*_CF | GACTGTTGGGCGGGTGTG |
| 132 | CEY17_00510*_CR | CATAAGGTGATGCAAGCGA |
| 133 | CEY17_12940*_CF | ACAAGATGGATAACAAGA |
| 134 | CEY17_12940*_CR | CCACTGAACTTCCTTGAC |
| 135 | CEY17_00330*_CF | CATAGCCAAACTCAGTATG |
| 136 | CEY17_00330*_CR | CACCATCACGTGAGGCTC |

### Example 4. Evaluation of L-valine production ability of a strain into which a variant polynucleotide has been introduced.

In order to evaluate the L-valine production ability of the recombinant strains constructed in Example 3-2, a 12-well plate assay was performed as follows.

First, each strain was inoculated into a 2 mL 96-deep well plate containing 0.35 mL of a seed medium, sealed with a gas-permeable cover, and shake-cultured at 30°C and 950 rpm for 20 hours. Thereafter, 0.02 mL of the seed culture was inoculated into a 10 mL 12-deep well plate containing 0.4 mL of a production medium, sealed with a gas-permeable cover, and shake-cultured at 30°C and 950 rpm for 60 hours. The compositions of the seed medium and the production medium are as follows.

### <Seed medium (pH 7.2)>

Glucose 50 g, peptone 10 g, yeast extract 10 g, urea 4 g, sodium chloride 2.5 g (based on 1 L of distilled water)

### <Production medium (pH 7.2)>

Glucose 100 g, ammonium sulfate 40 g, soybean protein 2.5 g, corn steep solids 5 g, urea 3 g, dipotassium phosphate 1 g, magnesium sulfate heptahydrate 0.5 g, biotin 100 µg, thiamine-HCl 1 mg, calcium pantothenate 2 mg, nicotinamide 3 mg, calcium carbonate 30 g

### (based on 1 L of distilled water)

The experiment was performed in triplicate. After completion of the culture, the L-valine production concentration was measured using high-performance liquid chromatography (HPLC), and the average values of the analysis results are shown in Table 7 below.

**[Table 7]**

| Strain name | L-valine concentration (g/L) | Relative concentration (%) compared to the parent strain |
|---|---|---|
| CJ7V | 2.10 | 100.0 |
| CJ7V△CEY17_06240::CEY17_06240* | 2.16 | 102.8 |
| CJ7V△CEY17_16000::CEY17_16000* | 2.11 | 100.6 |
| CJ7VΔCEY17_05275::CEY17_05275* | 2.12 | 101.0 |
| CJ7V△CEY17_06935::CEY17_06935* | 2.15 | 102.5 |
| CJ7VΔCEY17_04835::CEY17_04835* | 2.16 | 102.9 |
| CJ7VΔCEY17_06635::CEY17_06635* | 2.11 | 100.6 |
| CJ7V△Pn_CEY17_16100::Pn*_CEY17_16100 | 2.07 | 98.7 |
| CJ7V△Pn_CEY17_03290::Pn*_CEY17_03290 | 2.10 | 99.9 |
| CJ7V△Pn_CEY17_01650::Pn*_CEY17_01650 | 2.15 | 102.2 |
| CJ7V△Pn_CEY17_01655::Pn*_CEY17_01655 | 2.12 | 101.1 |
| CJ7V△Pn_CEY17_02820::Pn*_CEY17_02820 | 2.11 | 100.5 |
| CJ7V△Pn_CEY17_07095::Pn*_CEY17_07095 | 2.20 | 104.8 |
| CJ7V△Pn_CEY17_15935::Pn*_CEY17_15935 | 2.12 | 101.1 |
| CJ7V△Pn_CEY17_14380::Pn*_CEY17_14380 | 2.18 | 104.0 |
| CJ7V△Pn_CEY17_01395::Pn*_CEY17_01395 | 2.17 | 103.3 |
| CJ7V△Pn_CEY17_00355::Pn*_CEY17_00355 | 2.04 | 96.9 |
| CJ7V△Pn_CEY17_10495::Pn*_CEY17_10495 | 2.13 | 101.5 |
| CJ7V△Pn_CEY17_15010::Pn* CEY17_15010 | 2.14 | 101.9 |
| CJ7V△Pn_CEY17_06565::Pn* CEY17_06565 | 2.19 | 104.3 |
| CJ7V△Pn_CEY17_05270::Pn* CEY17_05270 | 2.16 | 102.6 |
| CJ7V△Pn_CEY17_06260::Pn* CEY17_06260 | 2.11 | 100.5 |
| CJ7V△CEY17_12995::CEY17_12995* | 2.10 | 100.1 |
| CJ7V△CEY17_15045::CEY17_15045* | 2.12 | 100.7 |
| CJ7V△CEY17_00510::CEY17_00510* | 2.10 | 100.2 |
| CJ7VΔCEY17_12940::CEY17_12940* | 2.09 | 99.6 |
| CJ7VΔCEY17_00300::CEY17_00300* | 2.11 | 100.2 |

As shown in Table 7, it was confirmed that most of the L-valine-producing strains into which the variant polynucleotides were introduced exhibited L-valine production ability equivalent to or greater than that of the parent strain. In particular, it was confirmed that 'CJ7V△Pn_CEY17_07095::Pn*_CEY17_07095', 'CJ7V△Pn_CEY17_14380::Pn*_CEY17_14380', and 'CJ7V△Pn_CEY17_06565::Pn*_CEY17_06565' showed an approximately 4% to 5% increase in L-valine production ability compared to the parent strain CJ7V.

Based on the above results, it was confirmed that L-valine can be produced more efficiently by introducing a variant of a promoter regulating the expression of the CEY17_07095 gene, and/or a variant of a promoter regulating the expression of the CEY17_14380 gene, and/or a variant of a promoter regulating the expression of the CEY17_06565 gene.

From the foregoing description, those skilled in the art to which the present disclosure pertains will appreciate that the present disclosure may be implemented in other specific forms without changing the technical spirit or essential features thereof. In this regard, the embodiments described above are to be understood in all respects as illustrative and not restrictive. The scope of the present disclosure should be construed as including all modifications or variations derived from the meaning and scope of the appended claims and their equivalents, rather than from the detailed description.

## Claims

1. A polynucleotide, comprising
a nucleotide sequence wherein the nucleotide at position 130 is substituted with thymine (T) in the nucleotide sequence of SEQ ID NO: 138,
a nucleotide sequence wherein the nucleotide at position 71 is substituted with adenine (A) in the nucleotide sequence of SEQ ID NO: 139, or
a nucleotide sequence wherein the nucleotide at position 150 is substituted with adenine (A) in the nucleotide sequence of SEQ ID NO: 140.

2. The polynucleotide according to claim 1, wherein the polynucleotide has a promoter activity.

3. The polynucleotide according to claim 1, wherein the polynucleotide has 80% or more and less than 100% sequence identity to the nucleotide sequences of SEQ ID NO: 138, SEQ ID NO: 139, or SEQ ID NO: 140.

4. The polynucleotide according to claim 1, wherein the polynucleotide comprises a nucleotide sequence of SEQ ID NO: 18, a nucleotide sequence of SEQ ID NO: 20, or a nucleotide sequence of SEQ ID NO: 25.

5. An expression cassette comprising the polynucleotide according to claim 1 and a target gene.

6. A microorganism of the genus *Corynebacterium*, comprising the polynucleotide according to any one of claims 1 to 4, or the polynucleotide and a target gene operably linked thereto.

7. The microorganism according to claim 6, wherein the microorganism of the genus *Corynebacterium* is *Corynebacterium glutamicum*.

8. A method for producing L-valine, comprising culturing the microorganism according to claim 6 in a medium.

9. The method for producing L-valine according to claim 8, further comprising recovering L-valine from the cultured medium or the microorganism.

10. Use of the microorganism according to claim 6 or 7 for producing L-valine.
